# EUROPEAN PATENT SPECIFICATION

(11) **EP 1 196 024 B1**
(45) Date of publication and mention of the grant of the patent: **05.10.2005**
(21) Application number: 00954546.8
(22) Date of filing: 20.07.2000
(51) Int. Cl.: A01H 5/10, A01H 4/00

(54) **NOVEL METHOD FOR THE GENERATION AND SELECTION OF TRANSGENIC LINSEED/FLAX PLANTS**
NEUES VERFAHREN ZUR HERSTELLUNG UND AUSWAHL VON TRANSGENEN FLACHSPFLANZEN UND LEINSAMEN
NOUVEAU PROCEDE PERMETTANT LA PRODUCTION ET LA SELECTION DE PLANTES DE LIN TRANSGENIQUES

(30) Priority: 20.07.1999 EP 99114074
(43) Date of publication of application: 17.04.2002
(73) Proprietor: GVS Gesellschaft für Erwerb und Verwertung von Schutzrechten MBH, 53115 Bonn (DE)
(72) Inventor: HEINZ, Ernst, D-22609 Hamburg (DE); SCHEFFLER, Jodi, Oxford, MS 38655 (US); VOSS, Hjördis, D-22761 Hamburg (DE)
(74) Representative: Vossius & Partner
(86) International application number: PCT/EP2000/006969
(87) International publication number: WO 2001/005221

(56) References cited:
- US-A- 5 750 870
- DATABASE BIOSIS [Online] BIOSCIENCES INFORMATION SERVICE, PHILADELPHIA, PA, US; AN 1996:406048, 1996 CHIKRIZOVA, O.F. ET AL.: "Optimization of conditions of long-fibred flax transgenic plants, resitant to chlorosulfon herbicide" XP002128297
- DATABASE BIOSIS [Online] BIOSCIENCES INFORMATION SERVICE, PHILADELPHIA, PA, US; AN 1981:135851, 1980 PRETOVA, A.: "Effect of Streptomycin on growth of immature flax linum-usitatissimum embryos" XP002128298

## Description

The present invention relates to a method for the generation and selection of transgenic plant cells and tissue as well as plants of the genus Linum. Furthermore, the present invention relates to transgenic plant cells, tissue and plants obtained by a method of the invention and their use in plant cell and tissue culture and plant breeding.

The plant family Linaceae comprises plants such as Linseed or Flax (Linum usitatissimum) one of the oldest cultivated plants. The bast fibers of the stem are used to prepare Flax and the seed for the preparation of Linseed oil. For example, the oil from flax seed is rich in the linolenic acid, and is used in the manufacture of paint products and for the production of linoleum. In spite of extensive work on protocols for the transformation of Flax, all attempts to produce transformed Flax plants by cultivation of hypocotyl segments with Agrobacterium and culturing the hypocotyl segments on plates containing optimized combinations of hormones and optionally on media containing an antibiotic for the initial selection of putative transformants remained unsatisfactory.
For all protocols except Bretagne-Sagnard et al. (Transgenic Research 5 (1996), 131-137), the *npt* II gene was used as the selectable marker and the antibiotic was kanamycin. In Bretagne-Sagnard, the selectable marker resistance gene was the aad with spectinomycin used as the selectable marker. For some protocols, further tests were carried out in a attempt to prove that the selected plants were not escapes, but were truly transformed. Unfortunately, these were usually only further tests for the selectable marker using the same antibiotic or PCR amplification. Often no Southern blot analysis was carried out and progeny were not produced or analyzed. The method of Lawrence et al. (Proc. 6th International Congress SABRAO (1989), 535-538), used cotyledons as the target tissue. McHughen and Jordan (Plant Cells Report 7 (1989), 611-614), also evaluated the use of cotyledons. They have so far been judged to be less efficient than hypocotyls as the target tissue. Another variation is a protoplast method described by Ling (Versuche zur Entwicklungsbiologie und somatische Genetik *in vitro* mit Arten der Gattung *Linum.* Dissertation des Doktorgrades, in der Mathematisch-Naturwissenschaftlichen Fakultät der Christian-Albrechts-Universität zu Kiel (1997)). Compared to the hypocotyl method, protoplasts produced significantly fewer transgenic plants, and those that were produced often exhibited morphological abnormalities. Zhan et al., Plant Molecular Biology 11 (1988), 551-559, used Agrobacterium rhizogenes instead of Agrobacterium tumefaciens as the Ti-plasmid carrier. A few transformed plants were obtained, but they all had morphological and/or physiological abnormalities.
Two groups reported on the production of some transformants. McHughen and co-workers have produced the flax variety Triffid (herbicide resistance, McHughen et al., Canadian J. of Plant Science 77 (1997), 641-643) while Ellis and co-workers (Ellis et al., Theoretical and Applied Genetics 85 (1992), 46-54), have developed transposon containing lines and introduced rust resistant genes. However, the results are only presented for one variety and the frequency of putative successful transformants is not given.
Hence, the production of flax primary transformants, if at all possible, seemed to be genotype-dependent, time-consuming and/or resulting initially in plants with aberrant morphology.

Thus, the technical problem underlying the present invention is to provide a reliable and efficient method for the generation and selection of stably transformed plants of the genus Linum.
The solution to this technical problem is achieved by providing the embodiments characterized in the claims.

Accordingly, the invention relates to a method for the generation of transgenic plants of the genus Linum comprising
(a) introducing a recombinant DNA molecule comprising at least one selectable marker gene which confers resistance to at least one antibiotic into plant cells;
(b) induction of transgenic callus from the cells of (a); and
(c) regeneration of transgenic plants from the induced callus, wherein
   (i) after callus induction and/or culturing the calli on a medium containing a first antibiotic
   (ii) the calli or shoots regenerated therefrom are transferred onto a medium containing a second antibiotic which is different from the first antibiotic.

Although techniques for the transformation and regeneration of transgenic plants of most dicotyledonous and monocotyledonous plants have been developed, thus far attempts to apply these protocols to plants of the family Linaceae, in particular flax were not successful. In accordance with the present invention, a new method has been developed which is based on the surprising finding that the application of two different antibiotics for the selection of putative flax transformants can be utilized for a new and reliable plant transformation, regeneration and selection process, which leads to transgenic plants which are phenotypically normal and fertile. In principle any basic medium known in the art may be used in the method of the invention, for example those described in Murashige and Skoog, Physiol. Plant 15 (1962), 473-497 and Gamborg, Exp. Cell Res. 50 (1968), 151-158.
One of the most important aspects of the method of the invention is to transfer growing calli or shoots derived therefrom successively from a medium containing a first antibiotic on a medium containing a second antibiotic which is different from the first one. Said first and second antibiotic may be detoxified by the gene product(s) of the same or different marker gene(s) present in the recombinant DNA molecule to be introduced in the method of the present invention. It is to be understood that the crucial step of the method of the present invention is step (c). Therefore, methods starting with plant material wherein plant cells have already been transformed with a recombinant DNA molecule as in step (a) and optionally callus formation has been induced are also encompassed by the present invention as long as step (c) described above and further explained below is performed.
Said method is suitable to introduce homologous and heterologous DNA sequences in the above-mentioned plants. The method of the invention allows studying the function and interaction of genes which are expressed in said plants. Thus, the method of the invention is particularly suited and useful for the engineering of transgenic plants, plant tissue and plant cells which preferably display improved properties upon transformation. The transformation method of the present invention has several advantages. For example, it is not necessary to carry out any pre-culture or to remove the epidermis from individual hypocotyl segments. Both of these procedures require more time and effort and according to the experiences of Mlynarova et al. (Plant Cell Reports 13 (1994), 282-285), neither is important for producing sufficient numbers of transformants. Furthermore, it is not necessary to transfer the callused hypocotyls to new selection plates or subculture the callus onto new selection plates. This was done in some form for all of the other procedures described in the prior art and resulted in a lengthening of the selection phase from three to six weeks. In accordance with the protocol of the present invention multiple harvests of the same plate can be made starting at three weeks until six weeks after the hypocotyls were first placed on the selection plates.
As described in detail further below in the present protocol, shoots emerging from the callus were transferred preferably directly to culture containers containing a second antibiotic to select among the regenerated shoots. All the other protocols used the same antibiotic for selection during the entire selection process. In the examples a two step process was used with kanamycin as the first antibiotic during the callus development and shoot initiation phase, and then G418 as the second antibiotic for the shoot selection phase.
In addition, preliminary tests can be used for the ability of the shoots to root on media containing G-418 and then to root on kanamycin plus carbenicillin. Ability to root on kanamycin is described as a sometimes successful indicator. In accordance with the present invention, it has been developed into a reliable indicator.
After two to three weeks, the rooted shoot can then be transferred to soil and used to produce seed for the next generation, thereby further shortening the time from the beginning of the transformation phase to the production of ripe seed.
Finally, the ability of a 2 mm segment, from the shoot stem, to produce green proliferating callus was tested. This was confirmed as a reliable indicator of successful transformation. These green calli could also quickly produce more shoots that could then be rooted and transferred to soil or used to perform further tests such as an ELISA assay or Southern blotting.
The above described "rooting test" and "green proliferating test" also represent important aspect of the present invention either alone or in combination.

In summary, with the method of the present invention, transformed rooted plants have been generated using three different commercial flax varieties. These plants are capable of flowering and have produced seed. The plants are morphologically similar to their wild-type control variety. Furthermore, three different genes were used to create several different promoter gene combinations. This indicates that the system is also robust and not strictly genotype dependent. In view of their economical importance flax or linseed are preferably used for the method of the present invention.

Preferred first and second antibiotics include kanamycin, paromycin, neomycin, gentamycin, G-418, streptomycin, spectinomycin and imidazole. The term "imidazole" used herein encompasses antibiotic or herbicide compounds that contain imidazole or imidazolinone as a reactive group. Selectable marker genes are preferably employed which encode neomycin phosphotransferase, streptomycin phosphotransferase or aminoglycoside-3-adenyltransferase or are genes conferring resistance to imidazole. Useful imidazole resistance genes are for example described in Hill (Biochem. J. 335 (1998), 653-661), Mourad (Mol. Gen. Genet. 243 (1994), 178-184), Zhu (Nat. Biotechnol. 18 (2000), 555-558), Burke (Mol. Gen. Genet. 242 (1994), 169-176) and Doignon (Plasmid 30 (1993), 224-233). Other suitable marker genes and corresponding agents for the identification and/or selection of transformed ceiis, callus and plants are described in the art; see De Block, EMBO J. 6 (1987), 2513-2518; Herrera-Estrella, EMBO J. 2 (1983), 987-995 and, for review Gelvin and Schilperoot, Plant Molecular Biology Manual. Kluver Academic Publishers Dordrecht, Boston, London (1988).

In a particular preferred embodiment of the present invention, said first antibiotic is kanamycin and said second antibiotic is G-418.
In one embodiment of the method of the present invention, the concentration of said first antibiotic is in the range of 150 to 200 mg/l. The optimal concentration depends on the flax variety used. For example, with the cultivar McGregor 200 mg/l are preferably used.
In a further embodiment of the method of the present invention, the concentration of said second antibiotic is 40 to 100 mg/l. The optimal amount depends on the variety and the phase of selection. For example, for the first transfer from the 1 st antibiotic to the 2nd with the variety McGregor 80 mg/l are preferably used.
As described in the appended examples the hypocotyl of plants is preferably used for the method of the present invention. The method of the present invention is preferably performed, wherein the plant cells, e.g., the hypocotyls of step (a) are derived from plants which are substantially at the same developmental state. This can be achieved by methods known in the art, for example the plants can be derived from synchronized germinating seeds.
In another preferred embodiment of the method of the inventions the DNA molecule is introduced by a method comprising:
(a) inoculation with Agrobacterium tumefaciens;
(b) particle bombardment; or
(c) microinjection.

Methods for the transformation using biolistic methods are well known to the person skilled in the art; see, e.g., Wan, Plant Physiol. 104 (1994), 37-48; Vasil, Bio/Technology 11 (1993), 1553-1558 and Christou (1996) Trends in Plant Science 1, 423-431. Microinjection can be performed as described in Potrykus and Spangenberg (eds.), Gene Transfer to Plants. Springer Verlag, Berlin, NY (1995).
Suitable strains of Agrobacterium tumefaciens and vectors are known to those skilled in the art and are described in the prior art (GV3101 (pMK90RK), Koncz, Mol. Gen. Genet. 204 (1986), 383-396; C58C1 (pGV 3850kan), Debleare, Nucl. Acid Res. 13 (1985), 4777; Bevan, Nucleic. Acid Res. 12(1984), 8711; Koncz, Proc. Natl. Acad.
Sci. USA 86 (1989), 8467-8471; Koncz, Plant Mol. Biol. 20 (1992), 963-976; Koncz, Specialized vectors for gene tagging and expression studies. In: Plant Molecular Biology Manual Vol 2, Gelvin and Schilperoort (Eds.), Dordrecht, The Netherlands: Kluwer Academic Publ. (1994), 1-22; EP-A-120 516; Hoekema: The Binary Plant Vector System, Offsetdrukkerij Kanters B.V., Alblasserdam (1985), Chapter V, Fraley, Crit. Rev. Plant. Sci., 4, 1-46; An, EMBO J. 4 (1985), 277-287).
Methods for the inoculation and incubation of the hypocotyls with Agrobacterium tumefaciens are well known in the art. In a preferred embodiment, the present invention relates to any one of the above described methods wherein the plant cell, plant tissue or plant is inoculated with *Agrobacterium tumefaciens* in the presence of a phenolic compound, preferably acetosyringone. Preferably, in particular when Agrobacterium tumefaciens is used for the transformation said recombinant DNA molecule comprises a binary vector.
The regeneration of transgenic plants from transformed plant tissue or cells requires changes of the concentration and ratio of auxin and cytokinin in order to induce callus and shoot development. This is preferably done by placing the tissue and cells or callus, respectively, successively on solid media which contain the appropriate hormones. In particular, the addition of cytokinins and auxins or substances which have the same biological function is required to promote cell division and, e.g., to generate plants from a single cell. Furthermore, concentrations of the two hormones may have to be changed during the different stages of cell division or organogenesis and varies for different plant species and varieties (Davies, (ed.) Plant Hormones. Physiology, Biochemistry and Molecular Biology. Dordrecht: Kluwer Academic Publishers (1995)). The use of appropriate hormone concentrations and the ratio of one to each other in the regeneration of transformed plants can be adjusted by the person skilled in the art, for example as described in the following embodiments or in the examples.

In a preferred embodiment of the method of the present invention, the medium containing said first antibiotic contains at least 0,05 mg/l auxin and at least 0,002 mg/l cytokinin.

In a further preferred embodiment of the method of the present invention said auxin is NAA and/or said cytokinin is TDZ and/or BAP.

In a particular preferred embodiment of the method of the present invention, the concentration of auxin and cytokinin is TDZ (0,002 mg/l) and NAA (0,05 mg/l) or BAP (2 mg/l) and NAA (0, 1 mg/l).
In a further preferred embodiment of the method of the present invention, said medium containing said second antibiotic is substantially free of auxins and/or cytokinins.

The culturing steps of the method of the present invention can be performed as follows.
The hypocotyls are cultured on a medium containing the hormones mentioned previously and the first antibiotic. A combination of antibiotics that can include carbenicillin and cefotaxime or ticarcillin/potassium clavulanate are added to eliminate Agrobacterium. The medium is also supplemented with Gamborgs B5 Vitamins.
The shoots are cultured on a medium with the second antibiotic and the same combination of antibiotics to eliminate Agrobacterium. The medium is also supplemented with the same vitamins, but at half of the concentration.
Shoots that remain green and grow are transferred to rooting medium with only the selection antibiotic and sometimes the auxin IBA (0.1 mg/l) to promote rooting.
Multiple shoots from the same original shoot are quickly produced by cutting leaf or steam segments from the original shoot and placing the segments on a medium that is similar to that used for the hypocotyls. Segments from shoots that are truly transformed and not "escapes", form callus which subsequently produces shoots. These shoots are put on rooting medium.

The media used in accordance with the method of the present invention comprise 2 to 10 g/l agar, most preferably 6 to 7 g/l. The appropriate amount of agar may also depend on the manufacturer. Results obtained in accordance with the present invention revealed that, for example, agar from Duchefa is advantageously used.

The method of the invention as described above and illustrated in the appended examples is generally applicable for the generation of transgenic plant cells, plant tissue and plants. The optimal conditions for callus growth and plant regeneration according to the method of the invention can be evaluated by changes in media composition within the above described embodiments. Since each cultivar or line may respond slightly different to changes in media composition, transformation and regeneration of transgenic plants according to the method of the invention may be expedited by testing the different lines/cultivars by the protocol as outlined in the examples.

In a further preferred embodiment of the above described invention the DNA molecule to be introduced into the plants comprises a non-coding region of a gene and/or a nucleotide sequence encoding a polypeptide, peptide antisense RNA, sense RNA, viral RNA or ribozyme. Due to the method of the present invention it is now possible to study and specifically influence the expression of a developmental, regulatory and/or structural gene and its gene product in plants of the genus Linum, for example, proteins involved the metabolism of plants which are either derived from said plant family or from any other organism, such as microorganisms, algae, animals, insects, viruses, etc. or for example from other plant species, preferably from monocotyledonous or dicotyledonous plants, in particular from any plant of interest in agriculture, horticulture or wood culture, such as crop plants, namely those of the family Poaceae, any other starch producing plants, such as potato, maniok or leguminous plants, oil producing plants, such as oilseed rape, linseed, etc., plants using a polypeptide as storage substances, such as soybean, plants using sucrose as storage substance, such as sugar beet or sugar cane, trees, ornamental plants, plants which are of medical relevance, for example plants containing alkaloids, flavonoids and the like,
Some examples for the (over) expression of homologous or heterologous genes and antisense inhibition and co-suppression aiming at manipulating certain metabolic pathways in transgenic plants are reviewed in Herbers (TIBTECH 14 (1996), 198-205).
Ribozymes of different kinds which are capable of specifically cleaving the (pre)-mRNA of a target gene are described in, e.g., EP-B1 0 291 533, EP-A1 0 321 201 and EP-A2 0 360 257. Selection of appropriate target sites and corresponding ribozymes can be done as described for example in Steinecke, Ribozymes, Methods in Cell Biology 50, Galbraith et al. eds Academic Press, Inc. (1995), 449-460. An example for ribozyme mediated virus resistance is described in Feyter (Mol. Gen. Genet. 250 (1996), 329-228). Thus, it is immediately evident to the person skilled in the art that the method of the present invention can be employed to produce transgenic plants of the genus Linum with any desired trait (see for review TIPTEC Plant Product & Crop Biotechnology 13 (1995), 312-397) and comprise (i) herbicide tolerance (DE-A-3701623; Stalker, Science 242 (1988), 419), (ii) insect resistance (Vaek, Plant Cell 5 (1987), 159-169), (iii) virus resistance (Powell, Science 232 (1986), 738-743; Pappu, World Journal of Microbiology & Biotechnology 11 (1995), 426-437; Lawson, Phytopathology 86 (1996), 56 suppl.), (iv) ozone resistance (Van Camp, BioTech. 12 (1994), 165-168), (v) improving the preserving of fruits (Oeller, Science 254 (1991), 437-439), (vi) improvement of starch composition and/or production (Stark, Science 242 (1992), 419; Visser, Mol. Gen. Genet. 225 (1991), 289-296), (vii) altering lipid composition (Voelker, Science 257 (1992), 72-74), (viii) production of (bio)polymers (Poirer, Science 256 (1992), 520-523), (ix) alteration of the flower color, e.g. by manipulating the anthocyanin and flavonoid biosynthetic pathway (Heidmann, Nature 330 (1987), 667-678, WO90/12084), (x) resistance to bacteria, insects and fungi (Duering, Molecular Breeding 2 (1996), 297-305; Strittmatter, Bio/Technology 13 (1995), 1085-1089; Estruch, Nature Biotechnology 15 (1997), 137-141), (xi) alteration of alkaloid and/or cardiac glycoside composition, (xii) inducing maintaining male and/or female sterility (EP-A1 0 412 006; EP-A1 0 223 399; WO93/25695) and (xiii) higher longlivety of the inflorescences/flowers, (xiv) chemical compounds of industrial interest such as modification of fiber composition (Grima-Pettenati, Somatic cell genetics and molecular genetics of trees (1996), Kluwer, Boston), (xv) alteration of pathways to produce specific chemical compounds of industrial interest (John, PNAS 93 (1996), 12768) including those that can be targeted to specific organelles (Rooijen, Bio/technology 13 (1995), 72-77), or (xvi) production of novel polyunsatured fatty acids (PUFAS). Furthermore, the method of the invention can be used for insertion mutagenesis using, for example, gene targeting vectors known in the art (see, e.g., Hayashi, Science 258 (1992), 1350-1353; Fritze and Walden, Gene activation by T-DNA tagging. In *Methods in Molecular* biology 44 (Gartland, K.M.A. and Davey, M.R., eds). Totowa: Human Press (1995), 281-294) or transposon tagging (Chandlee, Physiologia Plantarum 78 (1990), 105-115).
The DNA molecules used in the method of the invention may contain further functional elements, for example "left border"- and "right border"-sequences of the T-DNA of Agrobacterium which allow for stable integration into the plant genome. Furthermore, methods and vectors are known to the person skilled in the art which permit the generation of marker free transgenic plants, i.e. the selectable or scorable marker gene is lost at a certain stage of plant development or plant breeding. This can be achieved by, for example cotransformation (Lyznik, Plant Mol. Biol. 13 (1989), 151-161; Peng, Plant Mol. Biol. 27 (1995), 91-104) and/or by using systems which utilize enzymes capable of promoting homologous recombination in plants (see, e.g., WO97/08331; Bayley, Plant Mol. Biol. 18 (1992), 353-361); Lloyd, Mol. Gen. Genet. 242 (1994), 653-657; Maeser, Mol. Gen. Genet. 230 (1991), 170-176; Onouchi, Nucl. Acids Res. 19 (1991), 6373-6378). Methods for the preparation of appropriate DNA molecules are described by, e.g., Sambrook (Molecular Cloning; A Laboratory Manual, 2nd Edition (1989), Cold Spring Harbor Laboratory Press, Cold Spring Harbor, NY).
In a preferred embodiment the said non-coding region or said nucleotide sequence or the complementary strand thereof contained in the recombinant DNA molecule to be used in accordance with the method of the present invention is operatively linked to transcription and/or expression control sequences. Regulatory elements ensuring transcription and expression in prokaryotic and/or eukaryotic cells are well known to those skilled in the art and usually comprise promoters ensuring initiation of transcription and optionally poly-A signals ensuring termination of transcription and stabilization of the transcript. Additional regulatory elements may include transcriptional as well as translational enhancers. Such elements, e.g., the 3' region of the octopin synthasegene of agrobacteria are described in the prior art (see, e.g., Gielen, EMBO J. 8 (1989), 23-29).
For the transcription and/or expression in plant cells said non-coding region or said nucleotide sequence or the complementary strand thereof described are placed under the control of regulatory elements which may be heterologous or homologous with respect to the said non-coding region or said nucleotide sequence to be expressed as well with respect to the plant species to be transformed. In general, such regulatory elements comprise a promoter active in plant cells. To obtain expression in all tissues of a transgenic plant, preferably constitutive promoters are used, such as the 35 S promoter of CaMV (Odell, Nature 313 (1985), 810-812) or promoters of the polyubiquitin genes of maize (Christensen, Plant Mol. Biol. 18 (1982), 675-689). In order to achieve expression in specific tissues of a transgenic plant it is possible to use tissue and cell specific promoters (see, e.g., Stockhaus, EMBO J. 8 (1989), 2245-2251). Known are also promoters which are specifically active in seeds of different plants species, such as maize, Vicia, wheat, barley etc. Also microspore-specific regulatory elements and their uses have been described (WO96/16182). Inducible promoters may be used in order to be able to exactly control expression. An example for inducible promoters are the promoters of genes encoding heat shock proteins. Furthermore, the chemically inducible Tet-system may be employed (Gatz, Mol. Gen. Genet. 227 (1991), 229-237). Further suitable promoters are known to the person skilled in the art and are described, e.g., in Ward (Plant Mol. Biol. 22 (1993), 361-366).
In the case that the said nucleotide sequence is expressed in sense orientation it is in principle possible to modify the coding sequence in such a way that the polypeptide is located in any desired compartment of the plant cell. These include the endoplasmatic reticulum, the vacuole, the mitochondria, the plastides, the apoplast, the cytoplasm etc. Methods how to carry out this modifications and signal sequences ensuring localization in a desired compartment are well known to the person skilled in the art.
Preferred promoters are embryo specific promoters such as the napin or oleosin promoter. This embodiment is practically useful for manipulating the seed, for example in order to enhance and/or alter the oil content of the plant.
A corresponding strategy is the following. For example, the step in the biosynthetic pathway between linoleic acid (18:2) and linolenic acid (18:3) can be altered. Normal flax has approximately 58% linolenic acid, so it is already known that this reaction is preferred. Using a second highly expressed enzyme, it should be possible to increase the linolenic acid content by 10% to 25%. Said second gene can be derived from oilseed rape that codes for the delta 15 desaturase. This gene can be fully under the control of a strong seed specific promoter and transformed into flax. In seeds, the gene should be overexpressed and the percentage of linolenic acid increased. The GC analysis of flax seeds, for fatty acid content, was successful using either the same half seed technique developed for rape or by imbibing the seeds with water and cutting off the half of the cotyledon. The imbibed seeds were then germinated on filter paper and transferred to soil where they developed into normal plants. Transformation experiments were performed using the JH5 vector and either the flax variety Flanders or McGregor. The vector JH5 contains the Napin promoter from Brassica napus, the delta 15 desaturase and the thioesterase terminator.
In a further preferred embodiment, the method of the present invention is employed to modulate, preferably improve the quality of bast fibers in plants of the family Linaceae. This can be achieved for example, by the expression of enzymes involved in cellulose biosynthesis or their inhibition, for example by the expression of corresponding antisense RNAs. Examples for such enzymes include but are not limited to β-Glucan-β-Glucosyltransferase, sucrosesynthase, xylanase and other enzymes involved in the biosynthesis of the plant cell wall and polysaccharides. Similar approaches for genetically engineering plants for altered fibers which may be adapted to plants of the family Linaceae in accordance with the present invention are described in the prior art, for example for cotton plants in US-A-5,495,070, US-A-5,792,933, US-A-5,869,720. Promoters that are specifically expressed in bast fiber cells are preferably employed and are known to the person skilled in the art or can be easily identified and isolated by well established methods. For example, differential screening can be employed or the isolation of cDNAs specifically expressed in bast fibers. Such cDNAs can than be employed for screening of a genomic DNA library for the isolation of the regulatory sequences capable of tissue- and cell-specific expression of a heterologous DNA sequence in bast fiber cells. A corresponding approach is described, for example, in John, Proc. Natl. Acad. Sci. USA 89 (1992), 5769-5773.
Furthermore, it can be envisaged to genetically modify plants of the genus Linum in accordance with the method of the present invention such that pigments or colorants are intercalated in the bast fibers so as to sort of (pre)dye the bast fibers.

In a particularly preferred embodiment of the method of the invention the above described DNA molecule comprises a further selectable and/or scorable marker. Selectable marker genes useful for the selection of transformed plant cells, callus, plant tissue and plants are well known to those skilled in the art and comprise for example antimetabolite resistance as the basis of selection for dhfr, which confers resistance to methotrexate (Reiss, Plant Physol. (Life Sci. Adv.) 13 (1994), 143-149); npt, which confers resistance to the aminoglycosides neomycin, kanamycin and paromycin (Herrera-Estrella, EMBO J. 2 (1993), 987-995), hygro, which confers resistance to hygromycin (Marsh, Gene 32 (1984), 481-485), aad which confers resistance to spectinomycin (Svab, Plant Mol. Biol. 14 (1990), 197-205) and spt which confers resistance to streptomycin (Maliga, Mol. Gen. Genet. 214 (1988), 456-459). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the omithine decarboxylase inhibitor, 2-(difluoromethyl)-DL-omithine, DFMO (McConlogue, 1987, In: Current Communications in Molecular Biology, Cold Spring Harbor Laboratory ed.) or deaminase drom Aspergillus terreus which confers resistance to Blasticidin S (Tamura, Biosci. Biotechnol. Biochem. 59 (1995), 2336-2338).
Useful scorable markers are also known to those skilled in the art and are commercially available. Preferably said marker is a gene encoding luciferase (Giacomin, PI. Sci. 116 (1996), 59-72; Scikantha, J. Bact. 178 (1996), 121), green fluorescent protein (Gerdes, FEBS Lett. 389 (1996), 44-47) or β-glucuronidase (Jefferson, EMBO J. 6 (1987), 3901-3907).

As mentioned above the method of the invention can be used for the transformation and regeneration of substantially any plant of the genus Linum. Preferably, said plant is Linum usitatissimum, for example the commercial varieties McGregor, Flanders or Ed 45.

The method of the invention is in particular useful for the genetic manipulation of plant cells and plant tissue in order to obtain plants with modified, preferably with improved or useful phenotypes.
Thus, the present invention relates also to transgenic plant cells and callus obtainable according to the method of the invention which contain stably integrated into the genome a DNA molecule wherein said DNA molecule is foreign to the transgenic plant cell. By "foreign" it is meant that the DNA molecule is either heterologous with respect to the host cell, this means derived from a cell or organism with a different genomic background, or is homologous with respect to the host cell but located in a different genomic environment than the naturally occurring counterpart of said DNA molecule. This means that, if the DNA molecule is homologous with respect to the host cell, it is not located in its natural location in the genome of said plant cell, in particular it is surrounded by different genes or other genomic sequences. In this case the DNA molecule, i.e. the non-coding region or nucleotide sequence comprised therein may be either under the control of its own promoter or under the control of a heterologous promoter. The term "heterologous" with respect to the DNA molecule, i.e. the non-coding region or nucleotide sequence comprised therein being operatively linked to the promoter means that said non-coding region or nucleotide sequence is not naturally linked to the promoter. On the other hand, the non-coding region or nucleotide sequence comprised in the DNA molecule to be introduced in the plant according to the method of the invention may become under the control of an endogenous promoter of said plant, for example due to random insertion into the genome of said plant cell sufficiently adjacent to sequences capable of conferring transcription and optionally expression of said non-coding region or nucleotide sequence in said plant cell. In this respect, it is also to be understood that the DNA molecule introduced into the plant according to the method of the invention can be used to restore a mutant gene via homologous recombination (Paszkowski (ed.), Homologous Recombination and Gene Silencing in Plants. Kluwer Academic Publishers (1994)).
The presence and optionally expression of the DNA molecule, i.e. the non-coding region or nucleotide sequence comprised therein in the transgenic plant cells and plant tissue preferably leads to physiological and phenotypic changes in plants containing such cells, preferably such as described above.
Thus, the present invention also relates to transgenic plants comprising transgenic plant ceiis and tissue obtained according to the method of the invention.

In yet another aspect the invention also relates to harvestable parts and to propagation material of the transgenic plants obtainable according to the method of the invention or plant cells, tissue or a plant derived therefrom. Harvestable parts can be in principle any useful parts of a plant, for example, leaves, stems, fruit, seeds, roots etc. Propagation material includes, for example, seeds, fruits, cuttings, seedlings, tubers, rootstocks etc.

In another embodiment the present invention relates to the use of the components as defined in the method as described above for the generation of transgenic plants of the genus Linum. The use of the invention may be employed, for example, for promoting callus induction, organogenesis, shoot induction and/or root induction.

In a further embodiment the present invention relates to the use of transgenic plant cells, plant tissue and plants obtained according to the method of the invention for plant tissue culture and/or breeding. For example, the plant cells obtained according to the method of the invention can be used for protoplast fusion experiments. Furthermore, the transgenic plant cells, plant tissue and plants obtained according to the method of the invention can be used for a method for the identification of chemical and/or biological compounds, for example by contacting said transgenic plant cell, tissue or plant which preferably display a scorable and/or selectable phenotype due to the presence of a DNA molecule described above and introduced into said transgenic plant cell, tissue or plant according to the method of the invention with a (plurality of) compound(s) and determining those compounds which are capable of altering the scorable and/or selectable phenotype of said plant cells, tissue or plant. Said chemical or biological compounds may be (poly)peptides, nucleic acids, for example a cDNA expression library, antibodies, small organic compounds, hormones, peptidomimics, or PNAs (Milner, Nature Medicine 1 (1995), 879-880; Hupp, Cell 83 (1995), 237-245; Gibbs, Cell 79 (1994), 193-198). Furthermore, the present invention relates to the use of transgenic plant cells, plant tissue and plants obtained according to the method of the invention for the production of male and/or female sterility and for the alteration or modification of plant/pathogene interaction, in particular for the engineering of disease resistant plants. These goals can be achieved by strategies known in the art, see supra. The term "pathogen" includes, for example, bacteria, viruses, insects and fungi as well as protozoa. The method of the invention is also useful for the production of plants with modified fiber composition or with specific chemical or biological compounds produced tissue specifically.

The method of the invention can be used for the (improved) production of all kinds of transgenic plants of plant families hitherto not or less approachable for plant transformation and regeneration. The method of the present invention may be desirably applied to plants of the genus Linum, preferably flax, although other plant varieties are encompassed by the methods and uses described herein as well.

The figures show:
**Figure 1.** Shows (upper left) a plate with callus segments from hypocotyls that were incubated without Agrobacterium and regenerated without kanamycin, (middle left) a plate with callus segments from hypocotyls that were incubated without Agrobacterium, but regenerated with kanamycin, (upper and middle right) are calli from hypocotyls that were incubated with Agrobacterium containing the vector JH5 and regenerated with kanamycin. Lower photograph shows transformed shoots grown for 2 weeks an 60 mg/l G-418 compared with wild-type plants grown on 40 and 60 mgll for the same length of time.

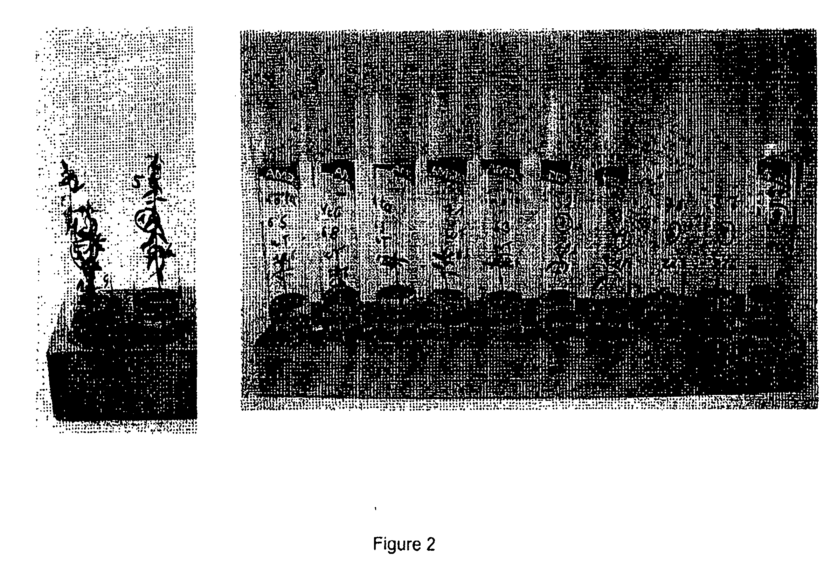

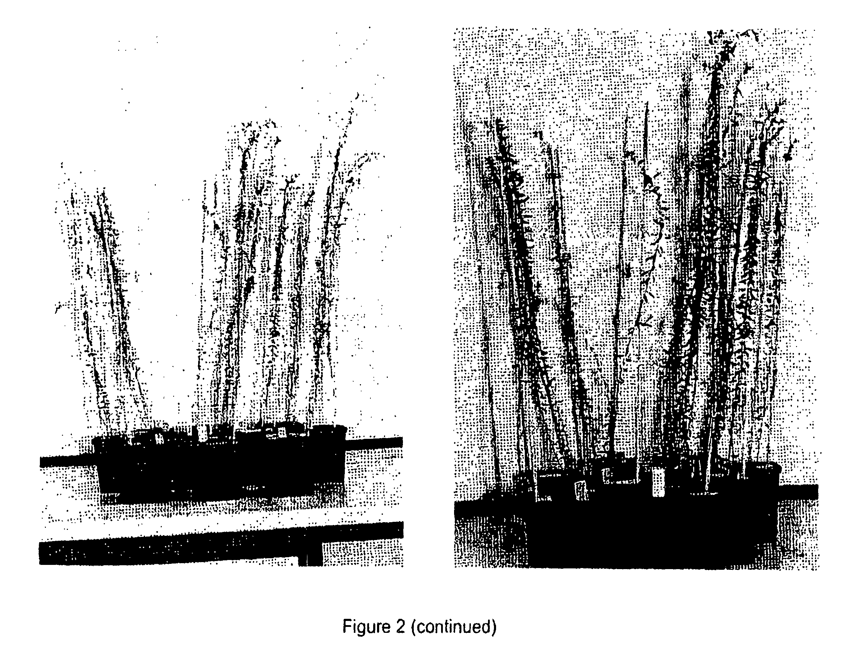

**Figure 2.** Shows (upper right) putative shoots in culture tubes, (upper left) rooted shoots ready to be transferred to soil, (lower left and right) confirmed transgenic plants (pink labels) compared to the wild-type control Flanders (white labels). All plants were phenotypically similar to the wild-type control plants.
**Figure 3.** Illustrates the structure of the JH5 binary vector described in Example 3.
**Figure 4.** Illustrates the structure of the pPZP911 binary vector described in Example 4.
**Figure 5.** Illustrates the structure of the pBI101.2 binary vector used in Example 5.
**Figure 6.** Illustrates the structure of the pHL9000 binary vector used in Example 6.
**Figure 7.** Gives a schematic map of the gene cassette used in Example 6.

The examples illustrate the invention.

### Example 1: Determining appropriate selection agents

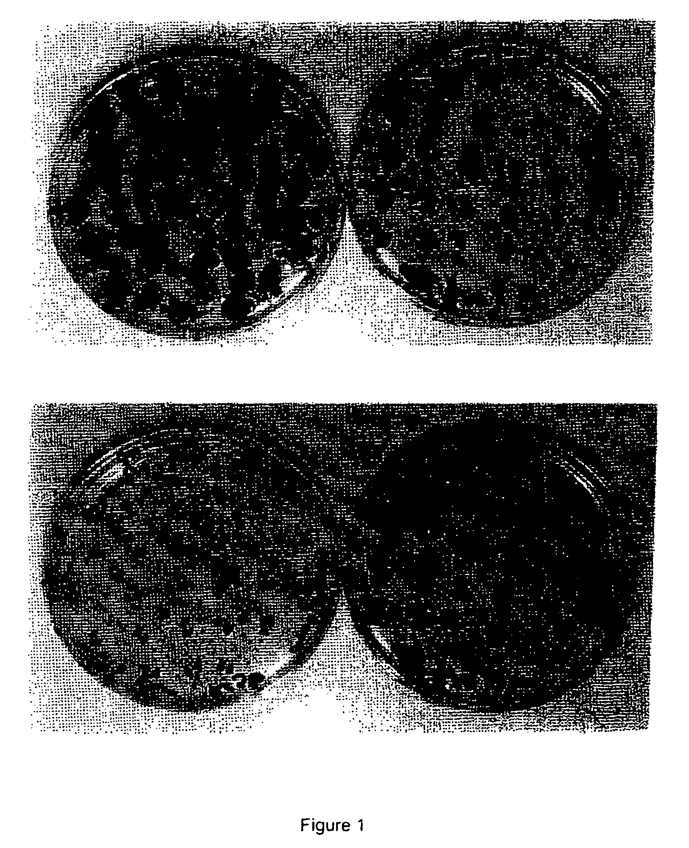

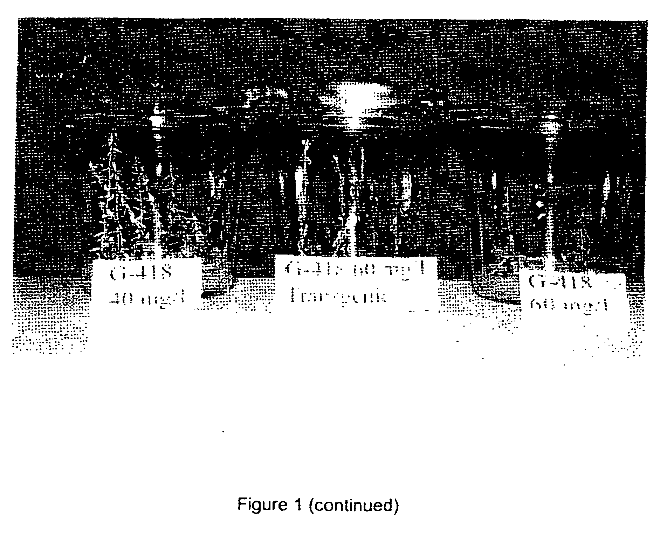

Tests done in accordance with the present invention indicated that hypocotyl segments used for the Agrobacterium tumefaciens inoculation, were sensitive to all the antibiotics tested except kanamycin. Even at low concentrations, the hypocotyls turned brown and failed to produce callus or shoots. Other preliminary tests indicated that although no callus was produced when G-418 was used as the selective antibiotic for the initial transformation, tests using regenerated shoots showed it had the advantage that wild-type flax had no natural tolerance for G-418. Subsequent tests showed that G-418 could be used as the selective agent for detecting transformed shoots in later rounds of selection (Table 1). Preferably, testing the two antibiotic system in the transformation experiments is done using kanamycin for the initial selection and G-418 for the second and third rounds of selection (Figure 1).

**Table 1.**

| **Ability of transgenic shoots and their wild-type counterparts to survive on medium with varying concentrations of G-418.** | | | | | | |
|---|---|---|---|---|---|---|
| **G-418** | **Flanders**^{**a**} | | **McGregor** | | **Ed 45** | |
| **mg/l** | **301**^{**b**} | **WT** | **324** | **WT** | **288** | **WT** |
| 0 | 4^{c} | 4 | 4 | 4 | 4 | 4 |
| | 4 | 4 | 4 | 4 | 4 | 4 |
| | | | | | | |
| 10 | 4 | 4 | 4 | 4 | 4 | 4 |
| | 4 | 4 | 4 | 4 | 4 | 4 |
| | | | | | | |
| 20 | 4 | 3 | 4 | 4 | 4 | 1 |
| | 4 | 4 | 4 | 3 | 4 | 2 |
| | | | | | | |
| 30 | 3 | 1 | 4 | 2 | 4 | 1 |
| | 4 | 3 | 4 | 2 | 4 | 2 |
| | | | | | | |
| 40 | 4 | 0 | 4 | 1 | 4 | 0 |
| | 4 | 1 | 4 | 0 | 4 | 0 |
| | | | | | | |
| 50 | 4 | 0 | 4 | 0 | 4 | 0 |
| | 4 | 0 | 4 | 0 | 3 | 0 |
| | | | | | | |
| 70 | 4 | 0 | 4 | 0 | 4 | 0 |
| | 4 | 0 | 4 | 0 | 4 | 0 |
| | | | | | | |
| 100 | 4 | 0 | 4 | 0 | 3 | 0 |
| | 3 | 0 | 4 | 0 | 4 | 0 |
| | | | | | | |
| 150 | 2 | 0 | 4 | 0 | 2 | 0 |
| | 4 | 0 | 3 | 0 | 0 | 0 |
| | | | | | | |
| 200 | 1 | 0 | 3 | 0 | 0 | 0 |
| | 2 | 0 | 1 | 0 | 1 | 0 |

| | | | | | | |
|---|---|---|---|---|---|---|
| a. For each combination of variety (Flanders, McGregor or Ed 45) and G-418 concentration, four shoots of a wild-type control and four shoots from a transformant were placed in one sterile container that was divided in half. | | | | | | |
| b. 301, 324 and 288 are all transformed with the JH5 vector described in Figure 3. | | | | | | |
| c. Number of shoots surviving 24 days on medium with G-418. | | | | | | |

### Example 2: General Linseed/Flax Transformation System

Sterilize seeds of the desired flax variety; for example: the flax variety Flanders

### Sterilization

50 min Ethanol
25 min 9% Hypochloride
4X rinse with bidistilled H₂O
IX 5 min in bidistilled H₂O
2X 15 min in bidistilled H₂O
Sow out the sterilized seeds on sterile medium, in culture containers that are covered so that light only comes in through the top of the container.

| 1 liter Seed Medium | |
|---|---|
| MS Medium | 4.4 g |
| myo-inositol | 400 mg |
| MES | 500 mg |
| Saccharose | 5 g |
| Agar | 7.8 g |

pH 6.2 and autoclave
Germinate under low light approximately 500 Lux for 6 to 7 days to get etiolated hypocotyls. On the day before beginning the co-cultivation with the Agrobacterium, start an overnight culture inoculated with the desired Agrobacterium-plasmid combination. YEP medium with the correct combination of antibiotics should be used. For example, for the PRE1 binary vector use spectinomycin 100 mg/l, streptomycin 200 mg/l and rifampicin 80 mg/l. On the next day, measure the OD₆₀₀, then centrifuge the culture and remove the supernatant. Resuspend the *Agrobacterium* pellet in Wash Medium

| 1 liter Wash Medium | |
|---|---|
| MS Medium | 4.4 g |
| myo-inositol | 400 mg |
| MES | 500 mg |

pH 5.8 and autoclave
Under sterile conditions, cut up the hypocotyls into 0.5 cm segments. Put hypocotyl segments in the *Agrobacterium* solution and incubate two hours. Transfer the hypocotyl segments onto co-cultivation Petri plates 94 x 16 mm (50 per plate) and incubate 4 days. Wash the hypocotyl segments in a solution of antibiotics to kill the *Agrobacterium*. For example, use Wash Medium containing carbenicillin 400 mg/l and 100 mg/l cefothaxime. Wash the segments 4 times and for 15 minutes each time. Transfer the washed hypocotyl segments to Petri plates 145 x 20 mm (50 per plate) containing an optimized combination of hormones, an antibiotic to select transformed cells and antibiotics to inhibit and kill any remaining *Agrobacterium*. For example:

| Selection Medium | |
|---|---|
| MS Gamborgs Medium | 4.4 g |
| myo-inositol | 400 mg |
| MES | 500 mg |
| Saccharose | 20 g |
| Agar | 7.8 g |

pH 5,8 and autoclave, after autoclaving add
BAP I mg/l
NAA 0.075 mg/l
carbenicillin 400 mg/l
cefotaxime 200 mg/l
kanamycin 160 mg/l

Put the Selection plates in a growth room with about 1000 Lux and temperature 230 to 26° C. It is important to regulate the conditions to avoid as much as possible excessive build-up of condensation on the lid of the Petri dishes. Leave the Selection plates in the growth room for six weeks and during that time cut off and transfer shoots as they appear. Shoots emerging from the callus are transferred directly to culture containers containing Shoot Medium.

| Shoot Medium | |
|---|---|
| MS Medium | 4.4 g |
| myo-inositol | 400 mg |
| MES | 500 mg |
| Saccharose | 15 g |
| Agar | 7.8 g |

pH 6.2 and autoclave, after autoclaving add 60 mg/l G-418, 400 mg/l carbenicillin and 100 mg/l cefotaxime.

For shoots that do not produce roots the first time on G-418 selection, but remain completely green and grow, the tip is cut off and transferred to a second culture container with G-418. Shoots producing roots under the G-418 Selection, are cut up and the tip transferred to a tube containing kanamycin 50 mg/l and carbenicillin 400 mg/l. Two 2 mm stem segments are transferred to a Selection plate (the same as used previously) and tested for green callus formation and subsequent shoot production. Shoots that test positive for these three tests can be immediately potted up. More shoots can also be quickly obtained from the callus test tissue and within a few weeks also potted up or used for further analysis by Southern blotting (Sambrook, Molec. Cloning a Laboratory Manual (1989), Cold Spring Harbor Laboratory Press) NPT II ELISA allay (5 Prime - 3 Prime Inc., Boulder Colorado USA).
Under the correct growth conditions, the plants will flower within 4 to 6 weeks depending on the variety. Seeds are fully ripe within 8 to 10 weeks. If the seeds must not be fully ripe, they can germinate and grow after 6 weeks with no problem and often earlier with special handling.

### Example 3: Experiment using the flax variety Flanders and the binary vector JH5

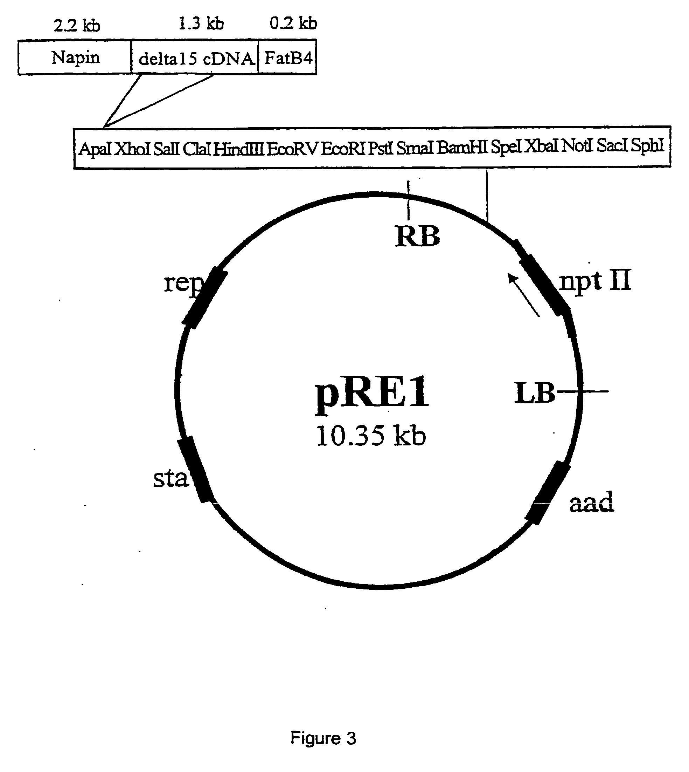

The binary vector JH5, used in this example, is shown in Figure 3 and was constructed as follows

The cDNA, coding for a delta-15 desaturase, was originally isolated from Brassica napus by Arondel (Science 258 (1992), 1353-1355) and obtained from the Arabidopsis Biological Resource Center (1060 Carmack Road, Columbus, Ohio USA). The plasmid was identified as pBNDES3. An XbaI/SalI fragment was excised from pBNDES3 and cloned into pTE200 digested with BamHI and Xhol. pTE200 is a pBluescript derivative (Martini, BioEngineering fuer Rapssorten nach Mass (1999), Vortraege fuer Pflanzenzuechtung Heft 45, pp. 133-154; Liddy Halm, Goettingen, Germany) that contains the promoter and terminator regions of an acyl-ACP thioesterase designated as FatB4. PTE200 was then digested with BspI20I and Smal, and a 2200 bp NotI/HindIII fragment of the Napin promoter (Scofield J., Biol. Chem. 262 (1987), 12202-12208) was inserted. The resulting plasmid was named JH3.
The fragment containing the Napin promoter, delta-15 cDNA and FatB4 terminator was excised by digesting with BspI20I and NotI and cloned into the Apal site of the binary vector pRE1 (Martini, BioEngineering fuer Rapssorten nach Mass (1999) Vortraege fuer Pflanzenzuechtung Heft 45, pp. 133-154; Liddy Haim, Goettingen, Germany). The resulting binary vector was designated JH5.
Regenerated plants were produced as described in Example 2. Hypocotyl segments, from 6 day old etiolated hypocotyls of the variety Flanders, were incubated for 1.5 hours in an Agrobacterium solution and them plated out on co-cultivation medium and left for 4 days. The JH5 vector was in the Agrobacterium strain C58 (Hood, J. Bacteriol. 168 (1986), 1291-1301). The negative control was hypocotyl segments incubated in Wash medium without Agrobacterium. After 4 days, the hypocotyl segments were transferred to selection plates and left for 6 weeks. The first shoots were removed from the selection plates 3 weeks later and the last after 6 weeks. All shoots were transferred to Sterile containers with medium containing G-418. Those shoots that produced roots or green callus on the stem end were transferred to tubes. Callus and rooting tests were performed as well as an NPT II ELISA assay. Rooted shoots were transferred to soil and allowed to produce seed (Table 2). A total of 1200 hypocotyl segments were inoculated with *Agrobacterium* and 26 putative transformants survived selection on G-418. After subsequent tests, 21 of the original 26 proved to be transformed (Table 2).

**Table 2.**

| **Shoots surviving after selection using kanamycin followed by G-418** | | | | | | | | | | | |
|---|---|---|---|---|---|---|---|---|---|---|---|
| | **Trfm Nr.** | **Plant ID** | **G-418 select. regime** | **root or green callus** | **Callus 1 2 1** | **Test** | **root** | **Test 2** | **NPT Test** | **Transfer to Soil** | **Seed Produced** |
| 1 | L-41 | 328 | 1 x 50mg/l | root | pos | pos | pos | pos | pos | yes | yes |
| 2 | L-41 | 329 | 1 x 50mg/l | root | pos | pos | pos | pos | pos | yes | yes |
| 3 | L-41 | 330 | 1 x 50mg/l | root | pos | pos | pos | pos | pos | yes | yes |
| 4 | L-41 | 331 | 1 x 50mg/l | root | pos | pos | pos | pos | pos | yes | yes |
| 5 | L-41 | 332 | 1 x 50mg/l | root | pos | pos | pos | pos | pos | yes | yes |
| 6 | L-41 | 333 | 1 x 50mg/l | green callus | - | neg | neg | neg | neg | no | no |
| 7 | L-41 | 334 | 1 x 50mg/l | green callus | neg | neg | neg | neg | neg | no | no |
| 8 | L-41 | 336 | 1 x 60mg/l | root | neg | neg | neg | neg | neg | yes | yes |
| 9 | L-41 | 337 | 1 x 60mg/l | green callus | neg | neg | neg | neg | neg | yes | yes |
| 10 | L-41 | 338 | 1 x 40mg/l | root | neg | neg | neg | neg | neg | yes | yes |
| 11 | L-41 | 341 | 2x 50&60mg/l | root | pos | pos | pos | pos | pos | yes | yes |
| 12 | L-41 | 342 | 2x 50&60mg/l | root | pos | pos | pos | pos | pos | yes | yes |
| 13 | L-41 | 343 | 2x 50&60mg/l | root | pos | pos | pos | pos | pos | yes | yes |
| 14 | L-41 | 344 | 2x 50&60mg/l | root | - | pos | pos | pos | pos | yes | yes |
| 15 | L-41 | 345 | 2x 50&60mg/l | root | pos | pos | pos | pos | pos | yes | yes |
| 16 | L-41 | 346 | 2x 50&60mg/l | root | pos | pos | pos | pos | pos | yes | yes |
| 17 | L-41 | 347 | 2 x 60mg/l | root | pos | pos | pos | pos | pos | yes | yes |
| 18 | L-41 | 348 | 2 x 60mg/l | root | pos | pos | pos | pos | pos | yes | yes |
| 19 | L-41 | 349 | 2 x 60mg/l | root | pos | pos | pos | pos | pos | yes | yes |
| 20 | L-41 | 350 | 2 x 60mg/l | root | pos | pos | pos | pos | pos | yes | yes |
| 21 | L-41 | 351 | 2 x 60mg/l | root | pos | pos | pos | pos | pos | yes | yes |
| 22 | L-41 | 352 | 2 x 60mg/l | root | pos | pos | pos | pos | pos | yes | yes |
| 23 | L-41 | 353 | 2 x 60mg/l | root | pos | pos | - | pos | pos | yes | yes |
| 24 | L-41 | 356 | 2 x 60mg/l | green callus | pos | pos | pos | pos | pos | yes | yes |
| 25 | L-41 | 358 | 2 x 60mg/l | root | pos | pos | pos | pos | pos | yes | yes |
| 26 | L-41 | 359 | 2 x 60mg/l | root | pos | pos | pos | pos | pos | yes | yes |

| Controls | | | | | | | | | | | |
|---|---|---|---|---|---|---|---|---|---|---|---|
| 27 | L-41 | WT1 | 1 x 0mg/l | not applicable | neg | neg | neg | neg | neg | yes | yes |
| 28 | L-41 | WT2 | 1 x 0mg/l | not applicable | neg | neg | neg | neg | neg | yes | yes |
| 29 | L-36 | 288 | 2 x 40mg/l | root | pos | pos | pos | pos | pos | yes | yes |
| 30 | L-37 | 301 | 2x 40&70mg/l | root | pos | pos | pos | pos | pos | yes | yes |
| 31 | L-39 | 324 | 2 x 40mg/l | root | pos | pos | pos | pos | pos | yes | yes |
| 32 | L-39 | 326 | 2 x 40mg/l | root | pos | pos | pos | pos | pos | yes | yes |

### Example 4: Experiment using the flax variety Flanders and the binary vector pPZPHV5

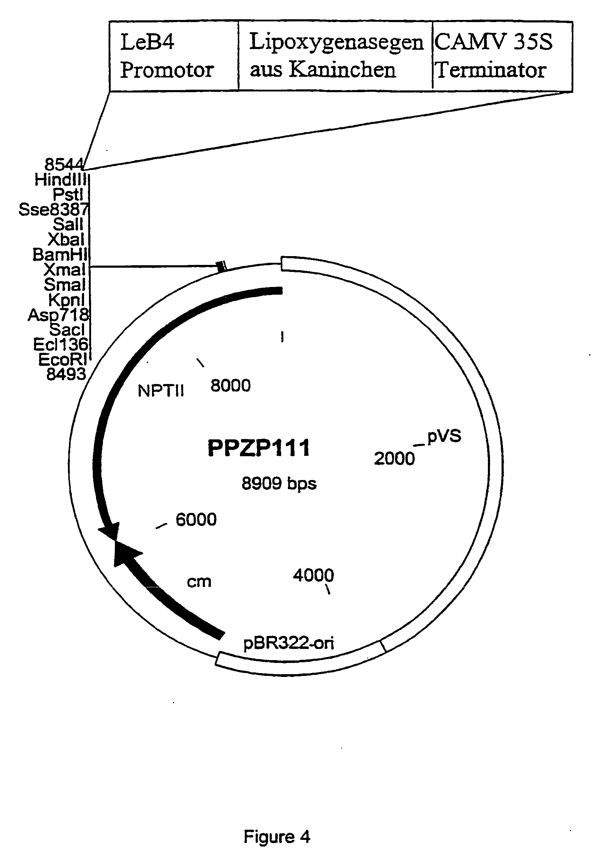

The binary vector pPZPHV5 contains a cDNA encoding a rabbit lipoxygenase under the control of the LeB4-promoter and the CaMV35S-terminator. The lipoxygenase from rabbit reticulocytes was originally isolated by Schewe et al. (Methods Enzymol. 71 (1981), 430-441). The plasmid pRL used therein is a derivative of the vector pBluescript II SK+. The cDNA encoding lipoxygenase was excised using the restriction endonucleases Sma I and Hind III, the protruding end of the Hind III digest was blunted and the thus obtained fragment was cloned into the vector pRT103-42-14 (Fiedler, 1996, Hochexpression rekombinanter Antikörperfragmente in transgenen Pflanzen; Dissertation zur Erlangung des Doktorgrades, in der Mathematisch-Naturwissenschaftlich-Technischen Fakultät der Martin-Luther-Universität Halle-Wittenberg) carrying the LeB4-promoter and the CaMV35S-terminator, said vector being cut using Sma I. From the resulting vector pRTHV5, the entire gene cassette was excised using Hind III and ligated into the binary vector pPZP111 (Figure 4; Hajdukiewicz et al., Plant. Mol. Biol. 25 (1994), 989-994). This construct was designated pPZPHV5.

Regenerated plants were produced as described in Example 3. Apart from the callus and the root tests, the plants were also tested by PCR in order to prove their transgenicity.

**Table 3.**

| **Shoots of one transformation event surviving after selection using kanamycin followed by G-418** | | | | | | | | | |
|---|---|---|---|---|---|---|---|---|---|
| | **Trfm Nr.** | **Plant ID** | **G-418 select. regime** | **root or green callus** | **Callus test** | **root test** | **PCR Test** | **Transfer to soil** | **Seed produced** |
| 1 | LF36 | 345 | 2x 60mg/l | root | pos | pos | pos | yes | yes |
| 2 | LF36 | 362 | 2x 60mg/l | green callus | pos | pos | pos | yes | not yet |
| 3 | LF36 | 363 | 2x 60mg/l | green callus | pos | pos | pos | yes | not yet |
| 4 | LF36 | 364 | 2x 60mg/l | root | pos | pos | pos | yes | not yet |
| 5 | LF36 | 365 | 2x 60mg/l | root | pos | pos | pos | yes | not yet |
| 6 | LF36 | 366 | 2x 60mg/l | green callus | pos | pos | pos | yes | not yet |
| 7 | LF36 | 367 | 2x 60mg/l | root | pos | pos | pos | yes | yes |
| 8 | LF36 | 368 | 2x 60mg/l | root | pos | pos | pos | yes | yes |
| 9 | LF36 | 374 | 2x 60mg/l | root | pos | pos | pos | yes | not yet |
| 10 | LF36 | 375 | 2x 60mg/l | green callus | pos | pos | pos | yes | yes |
| 11 | LF36 | 376 | 2x 60mg/l | root | pos | pos | pos | yes | yes |
| 12 | LF36 | 377 | 2x 60mg/l | root | pos | pos | pos | yes | not yet |
| 13 | LF36 | 378 | 2x 60mg/l | root | pos | pos | pos | yes | not yet |
| 14 | LF36 | 379 | 2x 60mg/l | root | pos | pos | pos | yes | not yet |
| 15 | LF36 | 386 | 2x 60mg/l | green callus | pos | pos | n.t. | yes | yes |
| 16 | LF36 | 387 | 2x 60mg/l | green callus | pos | pos | n.t. | yes | not yet |

| Controls | | | | | | | | | |
|---|---|---|---|---|---|---|---|---|---|
| 17 | LF36 | WT1 | 1x 0mg/l | not applicable | neg | neg | neg | yes | yes |
| 18 | LF36 | WT2 | 1x 0mg/l | not applicable | neg | neg | neg | yes | yes |

### Example 5: Experiment using the flax variety Flanders and the binary vector pJH7

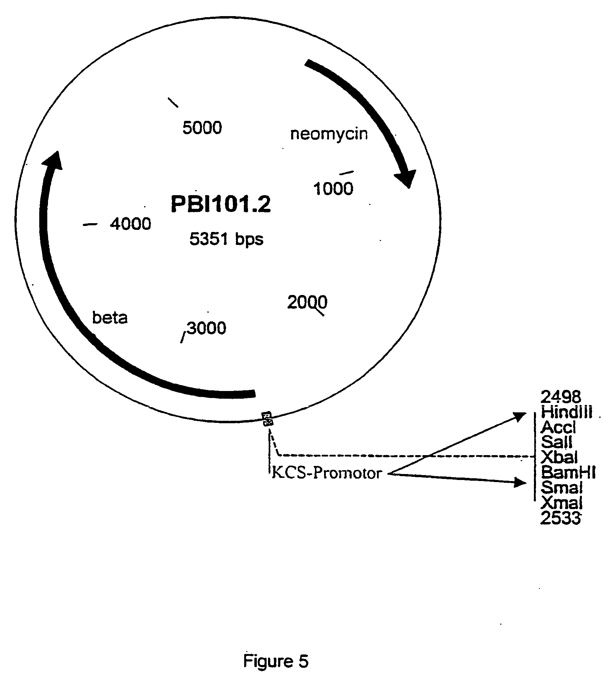

The binary vector pJH7 used in this example was generated by cloning into the binary vector pBI 101.2 (Figure 5), upstream of the GUS gene, the KCS-promoter of the gene encoding β-ketoacyl-CoA-synthase from *Brassica napus* (Han, 1999, β-Ketoacyl-CoA Synthase from *Brassica napus L.:* Functional Characterization and Promoter Analysis; Dissertation zur Erlangung des Doktorgrades am Fachbereich Biologie der Universität Hamburg). The cloning step has been carried out using the two restriction sites Sma I and Hind III. The resulting construct was designated pJH7.

Regenerated plants were produced as described in Example 3. Apart from callus and root tests, the plants were also tested by PCR in order to prove their transgenicity.

**Table 4.**

| **Shoots of one transformation event surviving after selection using kanamycin followed by G-418** | | | | | | | | | |
|---|---|---|---|---|---|---|---|---|---|
| | **Trfm Nr.** | **Plant ID** | **G-418 select. regime** | **root or green callus** | **Callus test** | **root test** | **PCR Test** | **Transfer to soil** | **Seed produced** |
| 1 | LF34 | 307 | 2x 60mg/l | green callus | pos | pos | pos | yes | yes |
| 2 | LF34 | 335 | 2x 60mg/l | green callus | pos | pos | pos | yes | yes |
| 3 | LF34 | 336 | 2x 60mg/l | green callus | pos | pos | n.t. | yes | not yet |
| 4 | LF34 | 338 | 2x 60mg/l | green callus | pos | pos | pos | yes | yes |
| 5 | LF34 | 340 | 2x 60mg/l | root | pos | pos | pos | yes | not yet |
| 6 | LF34 | 341 | 2x 60mg/l | green callus | pos | pos | pos | yes | yes |
| 7 | LF34 | 342 | 2x 60mg/l | green callus | pos | pos | pos | yes | yes |
| 8 | LF34 | 344 | 2x 60mg/l | green callus | pos | pos | pos | yes | yes |
| 9 | LF34 | 347 | 2x 60mg/l | green callus | pos | pos | pos | yes | yes |
| 10 | LF34 | 348 | 2x 60mg/l | green callus | pos | pos | pos | yes | yes |
| 11 | LF34 | 349 | 2x 60mg/l | green callus | pos | pos | pos | yes | yes |
| 12 | LF34 | 354 | 2x 60mg/l | green callus | pos | pos | pos | yes | yes |
| 13 | LF34 | 355 | 2x 60mg/l | green callus | pos | pos | pos | yes | yes |
| 14 | LF34 | 356 | 2x 60mg/l | green callus | pos | pos | pos | yes | yes |
| 15 | LF34 | 357 | 2x 60mg/l | green callus | pos | pos | pos | yes | yes |
| 16 | LF34 | 381 | 2x 60mg/l | green callus | pos | pos | pos | yes | not yet |
| 17 | LF34 | 382 | 2x 60mg/l | green callus | pos | pos | pos | yes | yes |
| 18 | LF34 | 383 | 2x 60mg/l | green callus | pos | pos | pos | yes | yes |
| 19 | LF34 | 384 | 2x 60mg/l | green callus | pos | pos | pos | yes | yes |
| 20 | LF34 | 385 | 2x 60mg/l | green callus | pos | pos | pos | yes | yes |

| Controls | | | | | | | | | |
|---|---|---|---|---|---|---|---|---|---|
| 21 | LF34 | WT1 | 1x 0mg/l | not applicable | neg | neg | neg | yes | yes |
| 22 | LF34 | WT2 | 1x 0mg/l | not applicable | neg | neg | neg | yes | yes |

### Example 6: Experiment using the flax variety Flanders and the binary vector pHLHVO

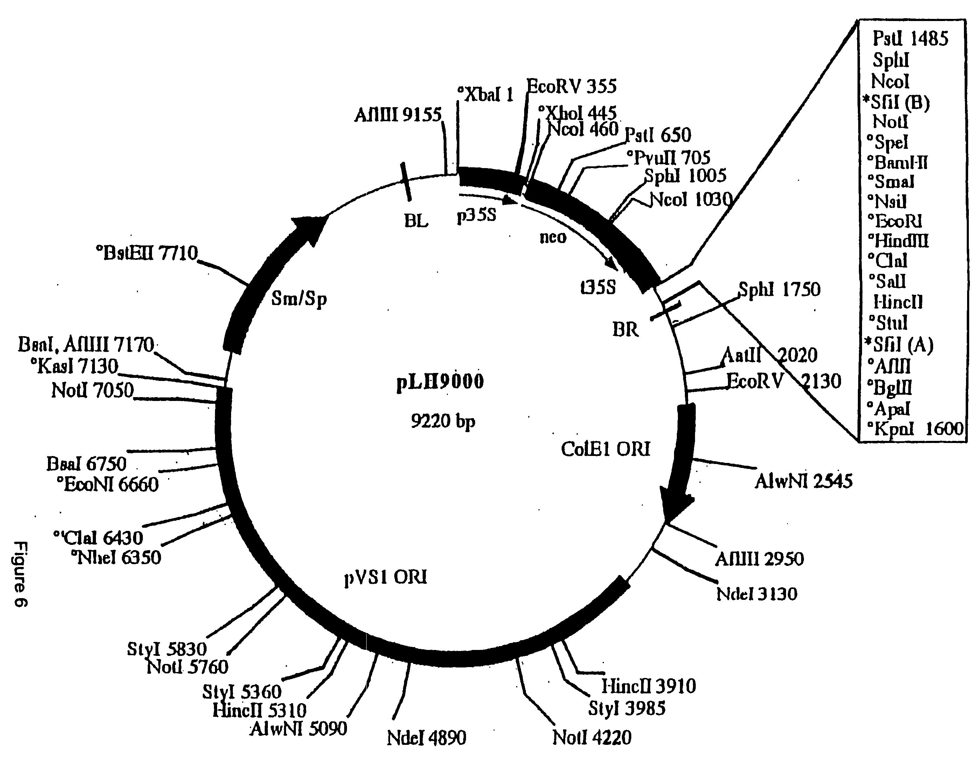

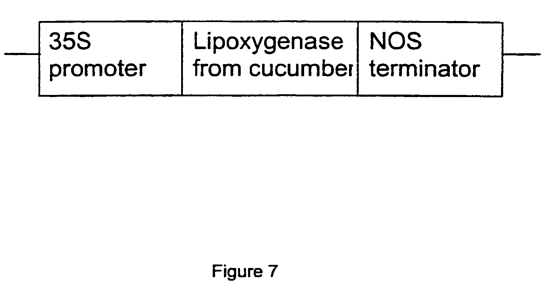

The binary vector pHLHVO is a derivative of the binary vector pHL9000 (Figure 6; Martini, BioEngineering für Rapssorten nach Maß (1999), Vorträge für Pflanzenzüchtung Heft 45, 155-171, Liddy Halm, Göttingen) into which the gene cassette shown in Figure 7 was cloned. The gene cassette was amplified via PCR using two specific primers and the vector pBI121-LBLOX (Hause et al., Planta 210 (2000), 708-714) as template. The sequence of the primer oligonucleotides are: Spe35: 5' act agt aga gga cct aac aga ac 3' (SEQ ID NO: 1); and

NoXho: 5' ctc gag cga tct agt aac ata gat gac 3' (SEQ ID NO: 2).

At the 5' end the primer Spe35 has a Spe I site and the primer NoXho a Xho I site (both marked by bold characters). Using these restriction sites, the gene cassette was cloned into pHL9000 which had been digested with Spe I and Sal I. The resulting construct was designated pHLHVO.

Regenerated plants were produced as described in Example 3. Apart from callus and root tests, the plants were also tested by PCR in order to prove their transgenicity.

**Table 5.**

| **Shoots of one transformation event surviving after selection with kanamycin followed by G-418** | | | | | | | | | |
|---|---|---|---|---|---|---|---|---|---|
| | Trfm Nr. | Plant ID | G-418 select. regime | root or green callus | Callus test | root test | PCR Test | Transfer to soil | Seed produced |
| 1 | LF49 | 443 | 1x 60mg/l | root | pos | pos | pos | yes | not yet |
| 2 | LF49 | 444 | 1x 60mg/l | root | pos | pos | pos | yes | not yet |
| 3 | LF49 | 449 | 1x 60mg/l | root | pos | pos | n.t. | yes | not yet |
| 4 | LF49 | 450 | 1x 60mg/l | root | pos | pos | pos | yes | not yet |

| **Controls** | | | | | | | | | |
|---|---|---|---|---|---|---|---|---|---|
| 5 | LF44 | WT1 | 1x 0mg/l | not applicable | neg | neg | neg | yes | not yet |
| 6 | LF46 | WT2 | 1x 0mg/l | not applicable | neg | neg | neg | yes | not yet |

### Legend for Tables 2 to 5

**Trfm Nr.** identifies from which experiment the transformant originated.
**Plant ID** is a unique number given to each putative transformant produced.
**G-418 select. regime** describes how many times the shoot was grown on G-418 before it was transferred to a tube and further tested.
**Root or green callus** indicates whether the shoot produced a root or only green callus when grown on G-418.
**Callus test** shows whether small stem sections taken from the shoot were able to enlarge and produce green callus. pos indicates that test was positive and neg indicates the test was negative
**Root test** tests the ability of the shoot to produce roots when grown on kanamycin.
**NPT II test** indicates the results of an NPT II ELISA assay carried out using the kit from 5 Prime - 3 Prime Inc. (Boulder Colorado USA)
**PCR test shows whether** a plant contains the npt II gene due to the presence of a specific PCR product.
**Transfer to soil** shows which transformants had shoots transferred to soil.
**Seed produced** indicates which of the shoots that were transferred to soil produced seed.
**Controls** WTI and WT2 are the variety Flanders that were regenerated, but not transformed with *Agrobacterium. 288* (Ed45), 301 (Flanders), 324 and 326 (McGregor) are transformants obtained in earlier experiments and previously confirmed by Southern blot analysis to be transgenic.

All reagents and containers used for this method can be obtained from the firm Duchefa (Haarlem, The Netherlands, e-mail DUCHEFA@WXS.NL)

## Claims

1. A method for the generation of transgenic plants of the family Linaceae comprising
(a) introducing a recombinant DNA molecule comprising at least one selectable marker gene which confers resistance to at least two antibiotics into plant cells;
(b) induction of transgenic callus from the cells of (a); and
(c) regeneration of transgenic plants from the induced callus, wherein
(i) after callus induction and/or culturing the calli on a medium containing a first antibiotic
(ii) the calli or shoots regenerated therefrom are transferred onto a medium containing a second antibiotic which is different from the first antibiotic.

2. The method of claim 1, wherein said plant is Linum usitatissimum.

3. The method of claim 1 or 2, wherein said plant is flax or linseed.

4. The method of any one of claims 1 to 3, wherein at least one of said first and second antibiotic are selected from the group consisting of kanamycin, paromycin, neomycin, gentamycin, G-418, streptomycin and spectinomycin.

5. The method of any one of claims 1 to 4, wherein said selectable marker gene encodes neomycin phosphotransferase, streptomycin phosphotransferase or aminoglycoside-3-adenyltransferase.

6. The method of any one of claims 1 to 5, wherein said first antibiotic is kanamycin and said second antibiotic is G-418.

7. The method of any one of claims 1 to 6, wherein the concentration of said first antibiotic is in the range of 150 to 200 mg/l.

8. The method of any one of claims 1 to 7, wherein the concentration of said second antibiotic 40 to 100 mg/l.

9. The method of any one of claims 1 to 8, wherein said plant cells are comprised in the hypocotyl of plants.

10. The method of claim 9, wherein said plants are derived from synchronized germinating seeds.

11. The method of any one of claims 1 to 10, wherein the recombinant DNA molecule is introduced by a method comprising:
(a) inoculation with Agrobacterium tumefaciens;
(b) particle bombardment; or
(c) microinjection.

12. The method of claim 11, wherein said inoculation with Agrobacterium tumefaciens is performed in the presence of acetosyringone.

13. The method of any one of claims 1 to 12, wherein said recombinant DNA molecule comprises a binary vector.

14. The method of any one of claims 1 to 13, wherein said medium containing said first antibiotic contains at least 0,05 mg/l auxin and at least 0,002 mg/l cytokinin.

15. The method of claim 14, wherein said auxin is NAA.

16. The method of claim 14 or 15, wherein said cytokinin is TDZ and/or BAP.

17. The method of any one of claims 14 to 16, wherein the concentration of auxin and cytokinin is TDZ (0,002 mg/l) and NAA (0,05 mg/l) or BAP (2 mg/l) and NAA (0,1 mg/l).

18. The method of any one of claims 1 to 17, wherein said medium containing said second antibiotic is substantially free of auxins and/or cytokinins.

19. The method of any one of claims 1 to 18, wherein the recombinant DNA molecule further comprises a nucleotide sequence encoding a polypeptide, peptide, antisense RNA, sense RNA, viral RNA or ribozyme.

20. The method of claim 19, wherein said nucleotide sequence is operatively linked to transcription and/or expression control sequences.

21. The method of any one of claims 1 to 20, wherein said recombinant DNA molecule comprises at least one further selectable and/or scorable marker gene.

22. Transgenic plant cells, tissue or a plant obtainable by the method of any one of claims 1 to 21 or plant cells, tissue or a plant derived therefrom comprising at least one recombinant DNA molecule comprising at least two selectable marker genes conferring resistance to at least two antibiotics.

23. Harvestable parts or propagation material of a plant of claim 22 comprising plant cells of claim 22.

24. Use of a recombinant DNA molecule as defined in any one of claims 1 to 20, Agrobacterium tumefaciens, antibiotics or hormones for the method of any one of claims 1 to 21.

25. Use of plant cells, plant tissue or plants of claim 22 for plant breeding, for a method for the identification of chemical and/or biological compounds, for the production of male and/or female sterile plants, disease-resistant plants or for plants with specific chemical or biological compounds produced tissue specifically.

## Patentansprüche

1. Verfahren zur Herstellung transgener Pflanzen der Familie Linaceae umfassend
(a) Einführen eines rekombinanten DNA-Moleküls, das zumindest ein selektierbares Markergen umfasst, das Resistenz gegen mindestens zwei Antibiotika vermittelt, in Pflanzenzellen;
(b) Induzieren von transgenem Kallus aus den Zellen von (a); und
(c) Regenerieren von transgenen Pflanzen aus dem induzierten Kallus, wobei
(i) nach Kallusinduktion und/oder Kultivieren der Kalli auf einem Medium, das ein erstes Antibiotikum enthält,
(ii) die Kalli oder Sprosse, die daraus regeneriert wurden, auf ein Medium transferiert werden, das ein zweites Antibiotikum enthält, das sich von dem ersten Antibiotikum unterscheidet.

2. Verfahren nach Anspruch 1, wobei die Pflanze Linum usitatissimum ist.

3. Verfahren nach Anspruch 1 oder 2, wobei die Pflanze Flachs oder Leinsamen ist.

4. Verfahren nach einem der Ansprüche 1 bis 3, wobei zumindest eines der ersten und zweiten Antibiotika aus der Gruppe bestehend aus Kanamycin, Paromycin, Neomycin, Gentamycin, G-418, Streptomycin und Spectinomycin ausgewählt ist.

5. Verfahren nach einem der Ansprüche 1 bis 4, wobei das selektierbare Markergen Neomycinphosphotransferase, Streptomycinphosphotransferase oder Aminoglycosid-3-adenyltransferase codiert.

6. Verfahren nach einem der Ansprüche 1 bis 5, wobei das erste Antibiotikum Kanamycin und das zweite Antibiotikum G-418 ist.

7. Verfahren nach einem der Ansprüche 1 bis 6, wobei die Konzentration des ersten Antibiotikum im Bereich von 150 bis 200 mg/l liegt.

8. Verfahren nach einem der Ansprüche 1 bis 7, wobei die Konzentration des zweiten Antibiotikum 40 bis 100 mg/l beträgt.

9. Verfahren nach einem der Ansprüche 1 bis 8, wobei die Pflanzenzellen im Hypocotyl der Pflanzen enthalten sind.

10. Verfahren nach Anspruch 9, wobei die Pflanzen von synchronisierten keimenden Samen stammen.

11. Verfahren nach einem der Ansprüche 1 bis 10, wobei das rekombinante DNA-Molekül durch ein Verfahren eingeführt wird, umfassend:
(a) Inokulieren mit Agrobacterium tumefaciens;
(b) Partikelbombardierung; oder
(c) Microinjektion.

12. Verfahren nach Anspruch 11, wobei die Inokulation mit Agrobacterium tumefaciens in Anwesenheit von Acetosyringon erfolgt.

13. Verfahren nach einem der Ansprüche 1 bis 12, wobei das rekombinante DNA-Molekül einen binären Vektor umfasst.

14. Verfahren nach einem der Ansprüche 1 bis 13, wobei das Medium, das das erste Antibiotikum enthält, mindestens 0,05 mg/l Auxin und mindestens 0,002 mg/l Cytokinin enthält.

15. Verfahren nach Anspruch 14, wobei das Auxin NAA ist.

16. Verfahren nach Anspruch 14 oder 15, wobei das Cytokinin TDZ und/oder BAP ist.

17. Verfahren nach einem der Ansprüche 14 bis 16, wobei die Konzentration von Auxin und Cytokinin TDZ (0,002 mg/l) und NAA (0,05 mg/l) oder BAP (2 mg/l) und NAA (0,1 mg/l) beträgt.

18. Verfahren nach einem der Ansprüche 1 bis 17, wobei das Medium, das das zweite Antibiotikum enthält, im wesentlichen frei ist von Auxinen und/oder Cytokininen.

19. Verfahren nach einem der Ansprüche 1 bis 18, wobei das rekombinante DNA-Molekül zusätzlich eine Nucleotidsequenz umfasst, die ein Polypeptid, Peptid, eine antisense-RNA, sense-RNA, virale RNA oder ein Ribozym codiert.

20. Verfahren nach Anspruch 19, wobei die Nucleotidsequenz mit Transkriptions- und/oder Expressionskontrollsequenzen funktionell verbunden ist.

21. Verfahren nach einem der Ansprüche 1 bis 20, wobei das rekombinante DNA-Molekül zumindest ein weiteres selektierbares und/oder quantifizierbares Markergen umfasst.

22. Transgene Pflanzenzellen, Gewebe oder eine Pflanze erhältlich durch das Verfahren nach einem der Ansprüche 1 bis 21 oder Pflanzenzellen, Gewebe oder eine Pflanze, die davon abgeleitet ist, umfassend zumindest ein rekombinantes DNA-Molekül, das mindestens zwei selektierbare Markergene umfasst, die Resistenz gegen mindestens zwei Antibiotika vermitteln.

23. Erntebare Teile oder Vermehrungsmaterial einer Pflanze gemäß Anspruch 22 umfassend Pflanzenzellen gemäß Anspruch 22.

24. Verwendung eines rekombinanten DNA-Moleküls wie in einem der Ansprüche 1 bis 20 definiert, Agrobacterium tumefaciens, Antibiotika oder von Hormonen für das Verfahren gemäß einem der Ansprüche 1 bis 21.

25. Verwendung von Pflanzenzellen, Pflanzengewebe oder Pflanzen nach Anspruch 22 zur Pflanzenzüchtung, für ein Verfahren zur Identifizierung von chemischen und/oder biologischen Verbindungen, zur Herstellung von männlich und/oder weiblich sterilen Pflanzen, krankheitsresistenten Pflanzen oder Pflanzen, die spezifische chemische oder biologische Verbindungen gewebsspezifisch herstellen.

## Revendications

1. Procédé pour la génération de plantes transgéniques de la famille Linaceae, comprenant :
(a) l'introduction d'une molécule d'ADN recombinante comprenant au moins un gène marqueur sélectionnable qui confère la résistance à au moins deux antibiotiques dans les cellules de plantes ;
(b) l'induction de calles transgéniques à partir des cellules de (a) ; et
(c) la régénération de plantes transgéniques à partir des cals induits, dans lequel,
(i) après l'induction de calles et/ou la culture des calles sur un milieu contenant un premier antibiotique
(ii) les calles ou pousses régérénées à partir de ceux-ci sont transférés dans un milieu contenant un deuxième antibiotique qui est différent du premier antibiotique.

2. Procédé selon la revendication 1, dans lequel ladite plante est un Linum usitatissimum.

3. Procédé selon la revendication 1, dans lequel ladite plante est du lin.

4. Procédé selon l'une des revendications 1 à 3, dans lequel au moins l'un desdits premier et second antibiotiques est choisi dans le groupe constitué de kanamycine, paromycine, néomycine, gentamycine, G-418, streptomycine et spectinomycine.

5. Procédé selon l'une des revendications 1 à 3, dans lequel ledit gène marqueur sélectionnable code une néomycine phosphotransférase, une streptomycine phosphotransférase, ou une aminoglycoside-3-adényltransférase.

6. Procédé selon l'une des revendications 1 à 5, dans lequel ledit premier antibiotique est la kanamycine et ledit second antibiotique est le G-418.

7. Procédé selon l'une des revendications 1 à 6, dans lequel la concentration dudit premier antibiotique est dans la plage de 150 à 200 mg/l.

8. Procédé selon l'une des revendications 1 à 7, dans lequel la concentration dudit second antibiotique est dans la plage de 40 à 100 mg/l.

9. Procédé selon l'une des revendications 1 à 8, dans lequel lesdites cellules de plantes sont comprises dans l'hypocotyle des plantes.

10. Procédé selon la revendication 9, dans lequel lesdites plantes sont dérivées à partir de graines en germination synchronisées.

11. Procédé selon l'une des revendications 1 à 10, dans lequel la molécule d'ADN recombinante est introduite par une méthode comprenant :
(a) l'inoculation avec Agrobacterium tumefaciens ;
(b) le bombardement de particules ; ou
(c) la micro-injection.

12. Procédé selon la revendication 11, dans lequel ladite inoculation avec Agrobacterium tumefaciens est réalisée en présence d'acétosyringone.

13. Procédé selon l'une des revendications 1 à 12, dans lequel la molécule d'ADN recombinante comprend un vecteur binaire.

14. Procédé selon l'une des revendications 1 à 13, dans lequel le milieu contenant ledit premier antibiotique contient au moins 0,05 mg/l d'auxine et au moins 0,002 mg/l de cytokinine.

15. Procédé selon la revendication 14, dans lequel l'auxine est la NAA.

16. Procédé selon la revendication 14 ou 15, dans lequel ladite cytokinine est TDZ et/ou BAP.

17. Procédé selon l'une des revendications 14 à 16, dans lequel la concentration d'auxine et de cytokinine est TDZ (0,002 mg/l) et NAA (0,05 mg/l) ou BAP (2 mg/l) et NAA (0,1 mg/l).

18. Procédé selon l'une des revendications 1 à 17, dans lequel ledit milieu contenant ledit second antibiotique est essentiellement dépourvu d'auxine et/ou de cytokinine.

19. Procédé selon l'une des revendications 1 à 18, dans lequel la molécule d'ADN recombinante comprend en outre une séquence nucléotidique codant un polypeptide, peptide, ARN antisens, ARN sens, ARN viral ou un ribozyme.

20. Procédé selon la revendication 19, dans lequel la séquence nucléotidique est liée de manière opérationnelle à des séquences de contrôle de la transcription et/ou de l'expression.

21. Procédé selon l'une des revendications 1 à 20, dans lequel la molécule d'ADN recombinante comprend au moins un gène marqueur sélectionnable et/ou mesurable supplémentaire.

22. Cellules ou tissu de plante transgénique, ou plante susceptible d'être obtenue par la méthode selon l'une quelconque des revendications 1 à 21, ou cellules ou tissu de plante ou une plante dérivés de ceux-ci, comprenant au moins une molécule d'ADN recombinante comprenant au moins deux gènes marqueur sélectionnables qui confèrent la résistance à au moins deux antibiotiques.

23. Parties ou matériel de propagation récoltables à partir d'une plante selon la revendication 22, comprenant des cellules de plante selon la revendication 22.

24. Utilisation d'une molécule d'ADN recombinante telle que définie dans l'une quelconque des revendications 1 à 20, d'Agrobacterium tumefaciens, d'antibiotiques et d'hormones pour la méthode selon l'une quelconque des revendications 1 à 21.

25. Utilisation de cellules de plantes, tissu de plante ou plantes selon la revendication 22 pour la sélection de plantes, dans une méthode d'identification de composés chimiques et/ou biologiques, pour la production de plantes à stérilité mâle et/ou femelle, de plantes résistantes à des maladies ou pour des plantes produisant des composés chimiques ou biologiques spécifiques de manière spécifique de tissus.
